(19) **Europäisches Patentamt**

**European Patent Office**

**Office européen des brevets**

(11) **EP 1 413 254 B1**

(12) **EUROPEAN PATENT SPECIFICATION**

(45) Date of publication and mention
of the grant of the patent:
**21.06.2006 Bulletin 2006/25**

(51) Int Cl.:
*A61B 10/00* (2006.01)  *G01N 11/00* (2006.01)

(21) Application number: **03023375.3**

(22) Date of filing: **11.10.1996**

(54) **Instrument for measuring saliva viscoelasticity to determine female fertile period**

Vorrichtung zur Messung der Viskoelastizität des Speichels zur Bestimmung der fruchtbaren Periode bei Frauen

Instrument pour mesurer la viscosité de la salive pour déterminer la période de fertilité de la femme

(84) Designated Contracting States:
**DE FR GB**

(43) Date of publication of application:
**28.04.2004 Bulletin 2004/18**

(62) Document number(s) of the earlier application(s) in accordance with Art. 76 EPC:
**96307427.3 / 0 835 636**

(73) Proprietor: **Kosasky, Harold J.**
**Chestnut Hill,**
**Massachusetts 02167 (US)**

(72) Inventor: **Kosasky, Harold J.**
**Chestnut Hill,**
**Massachusetts 02167 (US)**

(74) Representative: **Allsop, John Rowland**
**MacLeod Allsop**
**Island House**
**Lower High Street**
**Burford**
**Oxfordshire OX18 4RR (GB)**

(56) References cited:
**US-A- 4 167 110**   **US-A- 4 779 627**

**Description**

BACKGROUND OF THE INVENTION

Field of the invention

The present invention relates to the measurement of the viscoelasticity of saliva, and more particularly, to a device for the measurement of saliva viscoelasticity in order to determine a woman's fertile period.

The Prior Art

It has been known that the cervical mucus of a female has maximum fluidity just before ovulation, where ovulation is defined as the moment that an ovum is released from the follicle. This knowledge lead to the applicant's previous activities in the development of techniques for monitoring the viscoelasticity, or tackiness, and other properties of cervical mucus as a predictor of time of ovulation and to improvements in rheometer or viscometer apparatus for measuring such viscoelastic properties. See, for example, L.E. Kopito and H.J. Kosasky, "The Tackiness Rheometer Determination of the Viscoelasticity of Cervical Mucus" *Human Ovulation,* edited by E.S.E. Hafez, Elsevier, North-Holland Biomedical Press, 1979, pp. 351 et seq., and U.S. Patent Nos. 4,002,056 and 4,167,110. Though the viscoelasticity of the cervical mucus has several small dips in its characteristic curve of viscosity versus time preceding, during and following ovulation (a four day period), there is a distinct identifiable minimum viscoelasticity. Instruments designed to measure this effect are described in, for example, U.S. Patent Nos. 4,002,056 and 4,072,045.

Saliva is now known to undergo physiochemical changes during the menstrual cycle, including a change in its viscoelasticity. Especially pronounced is the change in viscoelasticity of sublingual saliva, the saliva found under the tongue. See, for example, S.S. Davis, "Saliva is Viscoelastic", Experientia, 26:1298, (1970), and R.H. Davis et al., "Saliva Viscosity Reflects the Time of Ovulation", Experientia, 30:911, (1974). As described in U.S. Patent No. 4, 779, 627, issued on October 25, 1988, to the present applicant, and entitled PROCESS AND APPARATUS FOR DETERMINING FEMALE OVULATION TIME BY MEASUREMENT OF SALIVA VISCOELASTICITY, the applicant previously discovered that sublingual saliva has a unique and reliably measurable minimum in viscoelasticity that is coincident with the ovulation cycle and its surge of estradiol.

There are devices on the market for measuring viscoelasticity to determine ovulation time, but these devices are designed to use cervical mucus as a sample medium, rather than saliva. The viscoelasticity of cervical mucus is an order of magnitude higher than that of saliva. So, devices designed to use cervical mucus as a sample medium are typically not sensitive enough to use saliva as a sample.

The above-identified Patent No. 4,779,627, in addition to disclosing a process for determining female ovulation time by measuring saliva viscoelasticity, discloses a device for measuring the viscoelasticity of the sublingual saliva. The device has a shape somewhat like a syringe, with an outer cup, an inner cup concentric with and located within the outer cup, and a plunger. A roughened surface on the end of the plunger holds the saliva sample. The plunger is inserted into the inner cup until the sample is compressed against the bottom of the inner cup. A predetermined amount of weight pulls the inner cup downward, stretching the sample. If the viscoelasticity of the saliva is low, the sample will fracture, causing the inner cup to fall to the bottom of the outer cup. An indicator at the bottom of the outer cup indicates that the inner cup has fallen to the bottom which, in turn, indicates that ovulation will soon take place. If however, the viscoelasticity of the saliva is high, the sample will hold the plunger and inner cup together s that the inner cup will not fall to the bottom, indicating that ovulation will not take place in the near future.

This device has several disadvantages. The first disadvantage is that the device can only be used conveniently for one person. The amount of weight that pulls the inner cup downward is selected for a specific person. There must have been a sublingual saliva sample measured from the same person at a time when the sublingual saliva is known to have the minimum viscoelasticity in order to select the amount of weight to calibrate the device.

The second disadvantage is that the device must be taken apart in order to take a sample. The plunger must be removed from the inner cup before being inserted in the mouth to obtain a saliva sample. This has the potential for the person to easily contaminate the sample by incorrectly reinserting the plunger after taking the sample, invalidating the measurement.

The force of adhesion of the sample to the surfaces between which it is compressed must be greater than the force of cohesion of the sample (the amount of force needed to fracture the sample), otherwise the sample will break away from the surface before it fractures, invalidating the measurement. In devices of the prior art, because adhesion is a function of the surface area but cohesion is not, the adhesion is made greater than the cohesion by enlarging the area of the opposing surfaces until the adhesion is greater. The problem is that the surface must be made so large that it cannot be used in a portable instrument.

Thus, there continues to be a need for a device for measuring the viscoelasticity of saliva to determine a female's ovulation time that is easy to use, does not have to be calibrated for an individual, and has a low chance of sample contamination.

## SUMMARY OF THE INVENTION

The object of the ovulation measuring device of the present invention is to overcome the disadvantages inherent in the devices of the prior art.

According to the present invention there is provided an instrument for determining female fertile period by measuring the viscoelasticity of saliva, said device comprising: an elongated sheath having a length along an axis and an opening at one extremity; a reciprocable escapement including a pair or elongated arms disposed approximately parallel to said axis, said arms having inner extremities within said sheath remote from said opening and outer extremities with mating surfaces; said escapement including a spring bias urging the separation of said mating surfaces from each other; said arms being mounted for relative movement of said mating surfaces among first relational positions at which said mating surfaces are separated, second relational positions at which said mating surfaces are in contact, and third relational positions at which said mating surfaces are free to separate under said spring bias; a control operatively connected to said escapement for optionally placing said mating surfaces into said first relational positions, and second relational positions, and said third relational positions; at least one of said mating surfaces being free to collect said saliva when said mating surfaces are in said first relational positions; said mating surfaces being constrained to compress said saliva therebetween when said mating surfaces are in said second relational positions; a timer for measuring time elapsed for separation of said surfaces when said surfaces are in said third relational positions; and said mating surfaces adapted to retain said saliva in a manner that causes said saliva to internally fracture before said saliva overcomes its adhesion to said mating surfaces when in said third relational positions.

The invention will now be described by way of example with reference to the accompanying drawings.

## BRIEF DESCRIPTION OF THE DRAWINGS

For a fuller understanding of the nature and object of the present invention, reference is made to the accompanying drawings, wherein:

Fig. 1 is a front elevational view of two plates embodying the present invention;
Fig. 2 is a front elevational view of the plates of Fig. 1 with a saliva sample;
Fig. 3 is a front elevational view of the plates of Fig. 1 pressed together;
Fig. 4 is a front elevational view of the plates of Fig. 1 separating after pressure is released;
Fig. 5 is a perspective view of a dual-prong embodiment of the instrument of the present invention;
Fig. 6 is a top view of the sheath of Fig. 5
Fig. 7 is a side-cross sectional view of the sheath of Fig. 6 taken along the line 7-7;
Fig. 8 is a top cross-sectional view of several components of the sheath of Fig. 1
Fig. 9 is an end view of the fork of Fig. 8;
Fig. 10 is an electrical schematic of the timing circuit;
Fig. 11 is a cross-sectional view of the stand of Fig. 5;
Fig. 12 is a side view of the escapement of Fig. 5;
Fig. 13 is a cross-sectional view of the first step of operation of the embodiment of Fig. 5;
Fig. 14 is a cross-sectional view of the second step of operation of the embodiment of Fig. 5;
Fig. 15 is a cross-sectional view of the third step of operation of the embodiment of Fig. 5;
Fig. 16 is a cross-sectional view of the fourth step of operation of the embodiment of Fig. 5;
Fig. 17 is a perspective view of a single-prong embodiment of the instrument of the present invention;
Fig. 18 is a front end cross-sectional view of the embodiment of Fig. 17;
Fig. 19 is a side cross-sectional view of the embodiment of Fig. 17;
Fig. 20 is a cross-sectional view of a portion of the embodiment of Fig. 17;
Fig. 21 is an electrical schematic of the timing circuit of the embodiment of Fig. 17;
Fig. 22 is a cross-sectional view of the second step of operation of the embodiment of Fig. 17;
Fig. 23 is a cross-sectional view of the third step of operation of the embodiment of Fig. 17;
Fig. 24 is a cross-sectional view of the fourth step of operation of the embodiment of Fig. 17;
Fig. 25 is a cross-sectional view of the fifth step of operation of the embodiment of Fig. 17;
Fig. 26 is a low-power microphotograph of a plate surface;
Fig. 27 is a high-power microphotograph of a plate surface;
Fig. 28 is an enlarged cross-section of a portion of a plate; and

DETAILED DESCRIPTION

The present invention includes several embodiments of an instrument for measuring saliva viscoelasticity and the components of the instrument that hold a saliva sample for use in the measurement.

Measuring Viscoelasticity

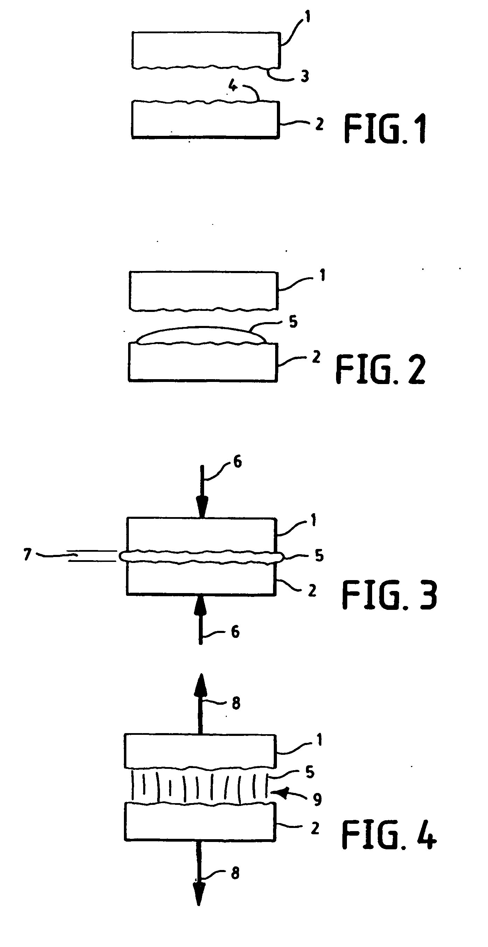

Figs. 1 to 4 show the process by which the viscoelasticity of saliva is measured. In Fig. 1, a pair of plates 1, 2 having sample surfaces 3, 4 are spaced apart. In Fig. 2, a saliva sample 5 is placed between the sample surfaces 3, 4. Typically, the saliva sample is taken from the saliva pool under the tongue, the majority of which is sublingual saliva, mixed with a small amount of submandibular saliva. In Fig. 3, the plates 1, 2 are pressed together with a predetermined force 6 until the sample surfaces 2, 3 are a predetermined distance apart 7. The predetermined distance 7 must be small enough so that the saliva sample 5 coats the entire area of the sample surfaces 3, 4, but large enough so that the saliva sample 5 is not squeezed out from between the sample surfaces 2, 3. In Fig. 4, the plates 1, 2 are pulled apart by a separation force 8 until the saliva sample fractures, as at 9. Fracturing occurs when the cohesion of the saliva sample 5 is overcome, where cohesion is defined as the tendency of parts of a body of like composition to hold together. The action represented by Fig. 4 implies two ways of measuring: (1) using a predetermined separation force 8 and measuring the time it takes for the saliva sample 5 to fracture, or (2) using a predetermined separation time and measuring the amount of separation force 8 needed to fracture the saliva sample 5.

The viscosity of a saliva sample 5 is a function of the separation force 8, the area of one of the sample surfaces 3, 4, and the amount of time that it takes for the sample surfaces 3, 4 to separate. These values are related by the following equation:

$$\text{viscosity} = \frac{\text{separation force/surface area}}{1/\text{separation time}} \qquad (1)$$

where the viscosity is calculated in poise (P), the separation force is measured in dynes (dy), the surface area is measured in square centimeters ($cm^2$), and the separation time is measured in seconds (s). The separation force/surface area term is also called the shear stress and the inverse of the separation time is also called the shear rate.

Note that equation (1) is one for viscosity, rather than for viscoelasticity. When using a Newtonian fluid, such as water, equation (1) will calculate pure viscosity. However, saliva is a non-Newtonian fluid. In a non-Newtonian fluid, there is an element of elastic recoil, or elasticity, along with the viscosity. Elasticity affects the separation time and separation force 8 of the plates 1, 2. Thus, the measurements used in equation (1) are affected by the elasticity of the saliva sample 5. Because there is no specific equation for viscoelasticity, the equation for viscosity is used, and the viscoelasticity is measured in viscosity-equivalent units, giving a Newtonian equivalent of the combination of viscosity and elasticity found in the non-Newtonian saliva sample 5.

The portions of the calculated viscoelasticity attributed to the viscosity and to the elasticity depend upon the thickness of the saliva (density, not breadth). As the thickness increases, the portion attributed to viscosity increases as a percentage of the viscoelasticity. For example, in a very thick fluid, the proportion of viscosity to elasticity may be 80% to 20%, while in a very thin fluid, the proportion may be 20% to 80%.

Another factor to consider is that, not only do the proportions of viscosity and elasticity change as a fluid thickens, but the absolute values of the viscosity and elasticity also change. For example, a thick fluid may have 80% of its viscoelasticity attributed to viscosity and 20% attributed to elasticity with absolute numbers of 64 poise attributed to viscosity and 16 poise attributed to elasticity, and a thin fluid may have 20% of its viscoelasticity attributed to viscosity and 80% attributed to elasticity with absolute numbers of 5 poise attributed to viscosity and 20 poise attributed to elasticity.

Measuring the viscoelasticity of a saliva sample 5 relies on the adhesion of the saliva sample 5 to the sample surfaces 3, 4, where adhesion is defined as the tendency, due to intermolecular forces, for matter to cling to other matter. In order to have a valid measurement, the force of adhesion of the saliva sample 5 to the sample surfaces 3, 4 must be greater than the force of cohesion of the saliva sample 5 so that the saliva sample 5 fractures before it separates from one of the sample surfaces 3, 4. Therefore, sample surfaces 3, 4 having a force of adhesion for the saliva sample 5 that is greater than the force of cohesion of the same saliva sample 5 must be provided.

The adhesion of the saliva sample 5 to a sample surface 3, 4 occurs over the entire area over which the saliva sample 5 and sample surface 3, 4 make contact. So, the larger the contact area, the proportionally greater will be the adhesion of the saliva sample 5 to the sample surfaces 3, 4.

The viscosity portion of the viscoelasticity measurement also increases in proportion to the amount of contact surface area, but because the viscosity is only part of the viscoelasticity measurement, the viscoelasticity only increases by an amount equal to the percentage that the viscosity is of the viscoelasticity. So, there is a point in the increase in surface area where the adhesion and cohesion forces are equal and any additional increase in surface area results in a greater adhesion than cohesion.

One way to increase the area of the sample surface 3, 4 is to increase the outer dimensions of the sample surface 3, 4. Another way is to roughen the surface, so that there are a plurality of valleys extending into the plates 1, 2. The contact area includes the area covered by the walls of any valleys extending into the sample surface 3, 4 to which the saliva sample can come into contact. Roughening the sample surfaces 3, 4 provides a greater contact area without increasing the outer profile of the plates 1, 2. This is a critical characteristic. The size of the instrument is no longer substantially dependent upon the size of the plates 1, 2, and can be made small enough, for example, to fit into a breast pocket.

Dual-Prong Instrument Embodiment

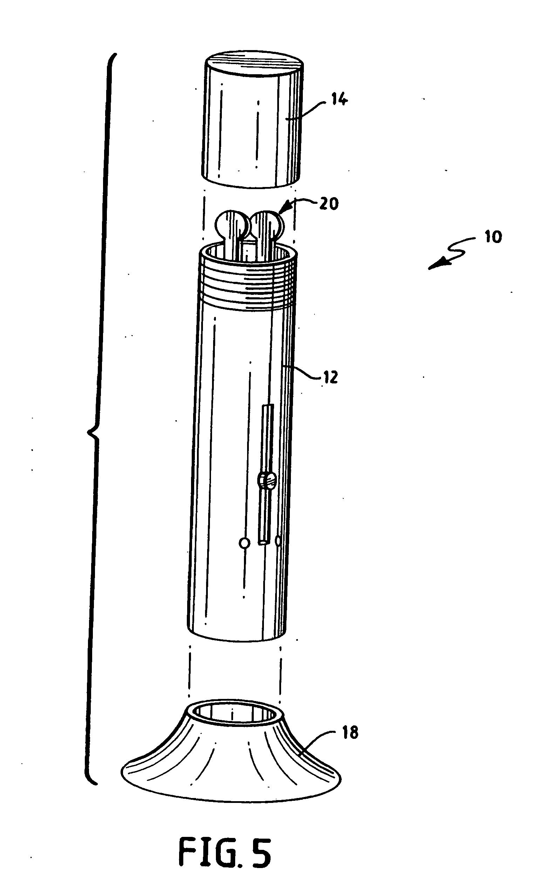

Fig. 5 shows the dual-prong embodiment of the instrument of the present invention 10. It is comprised of four separate components: the sheath 12, the cap 14, the stand 18, and the escapement 20.

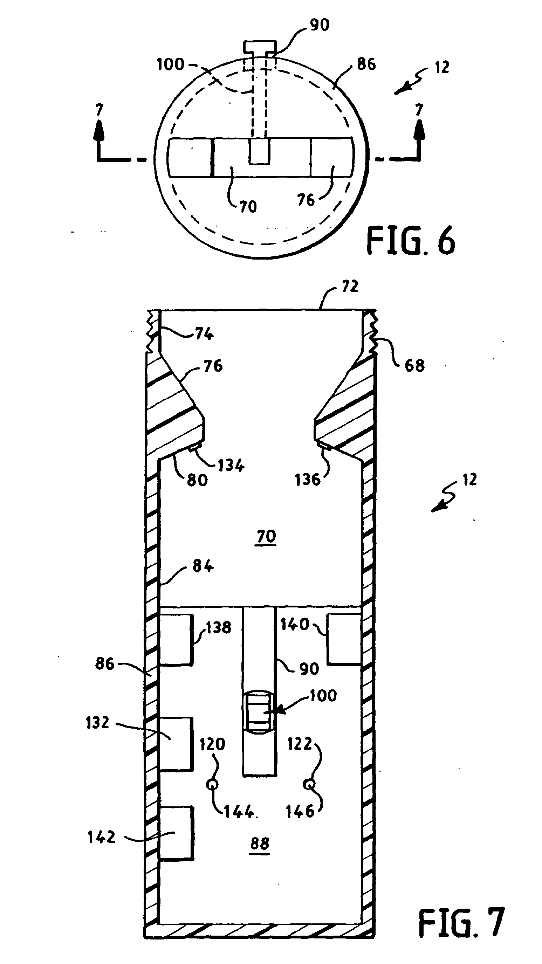

Figs. 6 and 7 show a top view and a side cross-sectional view, respectively, of the sheath 12. Preferably, the sheath 12 is composed of a rigid plastic and is shaped like a cylindrical cup, with a diameter of from 22 to 30 millimeters (mm) and a length of from 80 to 120 mm. In a preferred configuration, 5 mm of the outer surface of the sheath 12 adjacent to the open end 72 is threaded, as at 68. The remainder of the outer surface is approximately smooth. In another configuration, there is a annular protrusion near, but not adjacent to, the open end 72.

The central cavity 70, when viewed from the open end of the sheath 72, as in Fig. 6, is rectangular in shape and the rectangle is centered about the axis of the sheath 12. The narrow dimension of the cavity 70 is from 7 to 8 mm, and the wide dimension is approximately 18 mm at the open end 72 and for a distance of about 5 mm into the sheath 12, as at 74. Extending further into the sheath 12, the wide dimension of the cavity 70 decreases at an angle of about 45° to approximately 12 mm, as at 76, and remains substantially constant for a distance of from 3 to 5 mm, as at 78. The wide dimension increases at an angle of from 70° to 90° to approximately 18 mm, as at 80. The narrowing and widening of the cavity 70 defines a throat 82. The wide dimension of the cavity 70 continues to about 40 mm from the open end 72, as at 84, where the cavity 80 enlarges to a cylindrical shape 88, the thickness of the wall 86 being about 2 mm.

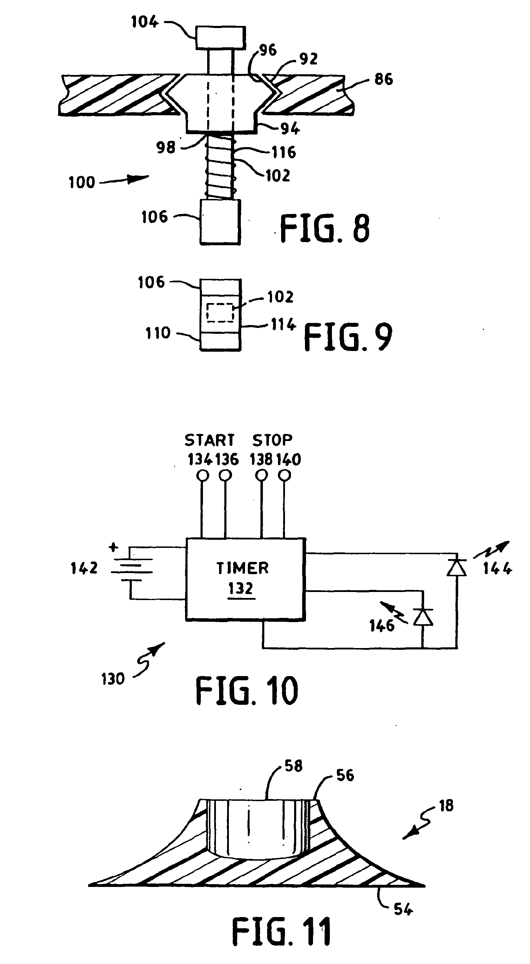

Within the wall 86 is a slot 90 that is 30 to 40 mm long and about 5 mm wide. As shown in Fig. 8, the edges of the slot 92 are formed into a "V" shape and a shuttle 94 with edges 96 adapted to mate with the slot edges 92 fits into the slot 90. Extending through the shuttle 94 and radially to the axis of the sheath 12 is a substantially rectangular bore 98. Located within the rectangular bore 98 is a fork 100. The center portion of the fork 102 has a substantially rectangular cross-section that is substantially the same size as the rectangular bore 98. The center portion 102 combined with the shape of the bore 98, allows the fork 100 to reciprocate radially within the bore 98 but prevents it from rotating within the bore 98. At the end of the center portion 102 outside the sheath 12 is a knob 104 that is large enough so that human fingers can push the fork 100 and shuttle 94 combination lengthwise within the slot 90 and can push and pull the fork 100 into and out of the shuttle bore 98. As in Fig. 9, at the end of the center portion 102 inside the sheath 12, there is a rectangular prong plate 114 perpendicular to the center portion 102. Extending from the upper and lower edges of the prong plate 114, parallel to and away from the center portion 102 are two prongs 106, 110. The length of the prongs 106, 110 is such that, when the fork 100 is pulled out of the shuttle bore 98, the ends of the prongs 106, 110 are not visible when looking from the open end 72 into the cavity 70. Surrounding the center portion 102 is a coil spring 116. The coil spring 116 biases the fork 100 into the sheath 12.

Straddling the slot 90 are two holes 120, 122 in the wall 86. Mounted within these holes 120, 122 are light-emitting diodes (LEDs) 144, 146 for indicating the result of the test performed by the device. The radiating surface of the LEDs faces outside the sheath 12.

Within the sheath 12 is a timing circuit 130, a schematic diagram of which is shown in Fig. 10. The timing circuit 130 is composed of a timer 132, a pair of start panels 134, 136, a pair of stop panels 138, 140, a battery 142, and the two LEDs 144, 146. When the start panels 134, 136 are electrically connected together, as described below, the timer 132 begins timing. When the stop panels 138, 140 are electrically connected together, as described below, the timer 132 stops timing. The timer 132 then compares the elapsed time to a predetermined value, and if the elapsed time is greater, it energizes one of the LEDs 144 momentarily, otherwise it energizes the other LED 146 momentarily. The battery 142 supplies electrical power to the timing circuit 130.

In an alternative configuration, the timer 132, battery 142, and LEDs 144, 146 are mounted on the outside of the sheath 12 in order to be more easily accessible.

The cap 14 is shaped substantially like an inverted cup that is 60 to 80 mm long and composed of a rigid plastic. In the preferred configuration, the inner surface of the open end 16 is threaded, where the threads are adapted to the outer threads of the sheath 68. In an alternate configuration, there is an annular depression about the inner surface of the open end of the cap that is adapted to mate with the annular protrusion of the sheath. The cap is attached by pressing the cap into the sheath until the protrusion snaps into the depression.

The stand 18, shown in cross-section in Fig. 11, holds the sheath 12 in an upright position. In the preferred embodiment, the base of the stand 54 is substantially circular and is about 100 mm in diameter. The side wall slopes upwardly and inwardly to a substantially flat top 56. The top 56 is substantially circular. The height of the stand is approximately 50 mm. Extending about 40 mm into the top 56 is a substantially cylindrical opening 58. The diameter of the opening 58 is substantially the same as the diameter of the sheath 12. The stand 18 is composed of an elastomeric material, such as polyurethane.

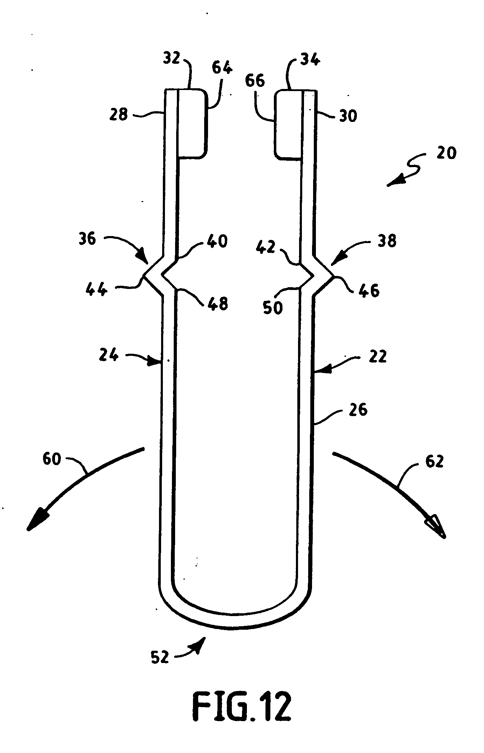

As shown in Fig. 12, the escapement 20 is a substantially elongated U-shaped strip of plastic or composite 22 that is coated with an electrically-conductive material. In an alternate configuration, the strip 22 is an electrically-conductive plastic or composite. The strip 22 is from 7 to 8 mm wide and about 2 mm thick. The width prevents the arms 24, 26 of the escapement 20 from twisting longitudinally in normal use. Approximately 35 mm from the outer extremity of each arm 24, 26 is a protrusion 36, 38. The protrusion 36, 38 is created by three bends in the strip 22. The upper bend 40, 42 and the lower bend 48, 50 angle approximately 45° outwardly from the plane of the arm 24, 26, forming the center bend of approximately 90°.

The cross portion of the escapement 20 is at the inner extremities of the arms 22, 24. The curve of the cross portion forms a spring 52, which forces the arms 24, 26 to pivot outwardly from a vertical position to a horizontal position, as at 60, 62. The amount of force exerted by the spring 52 is dependent on the material of which the strip 22 is composed and the thickness of the strip 22. The preferred force is described below.

In one embodiment of the escapement 20, at the outer extremity of each arm 24, 26 is a frame 28, 30 into which are permanently mounted plates 32, 34 by a substantially waterproof adhesive. In a second embodiment, the plates 32, 34 are removably mounted so that the plates 32, 34 may be discarded and replaced. In a third embodiment, the plates are integrally formed with the arms 24, 26. The plates 32, 34 have mating surfaces 64, 66, which are detailed below.

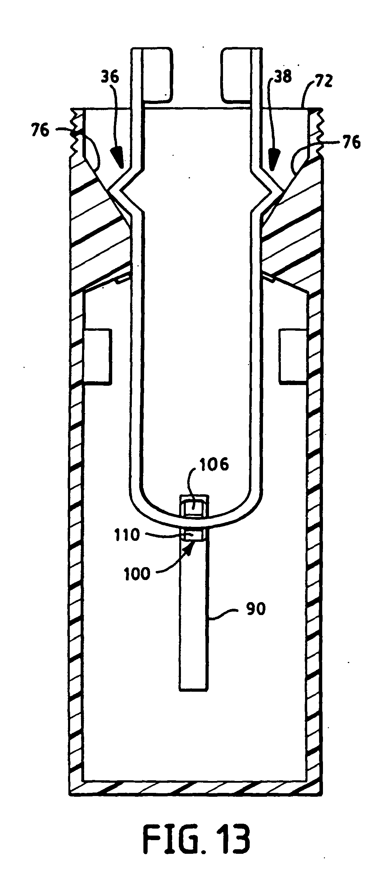

Figs. 13-16 detail, in cross-section, the internal operation of the dual-prong embodiment 10. Initially, the fork 100 is pushed to the end of the slot 90 nearest the open end of the sheath 72 and then pulled radially out of the sheath 12 by the knob 104 until the fork 100 is stopped by the prong plate 114. Because of the coil spring 116, the fork 100 must be held out of the sheath 12. As in Fig. 13, the escapement 20 is inserted into the open end 72 until the protrusions 36, 38 are resting on the upper throat surface 76. The knob 104 is released so that the coil spring 116 can force the fork 100 back into the sheath 12, after which the upper prong 106 is located just above the spring 52, and the lower prong 110 is located just below the spring 52. In this position, the fork 100 controls the movement of the escapement 20.

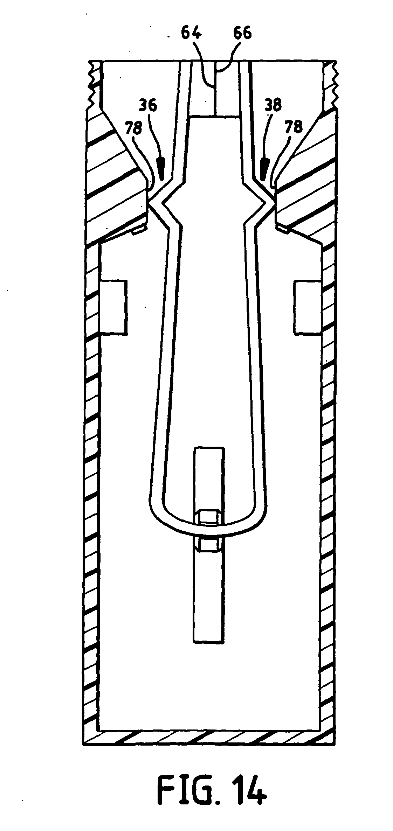

In Fig. 14, the operator has pushed the knob 104 away from the open end 72 a short distance. The action of the throat surface 78 on the protrusions 36, 38 is a camming mechanism that causes the mating surfaces 64, 66 to make flush contact. This action causes the upper portions of the escapement legs 22, 24 to deform outwardly at the protrusions 36, 38. The amount of force holding the mating surfaces 64, 66 and, as a consequent, compressing the saliva sample, is related to the amount of protrusion deformation. Preferably, the amount of force between the mating surfaces 64, 66 is approximately 15 grams.

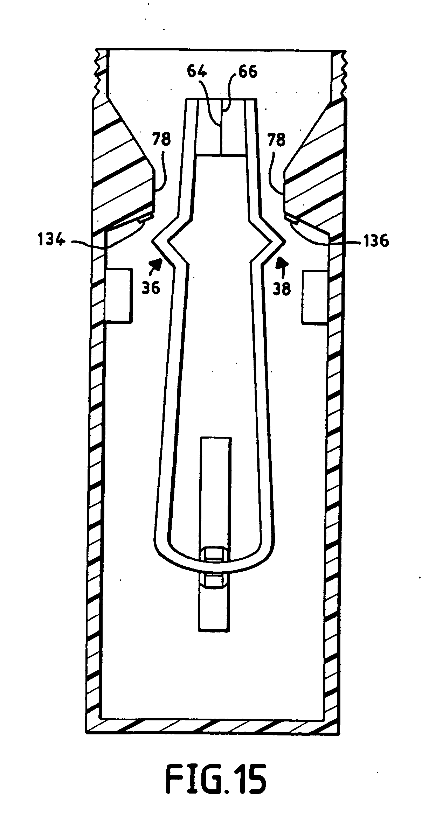

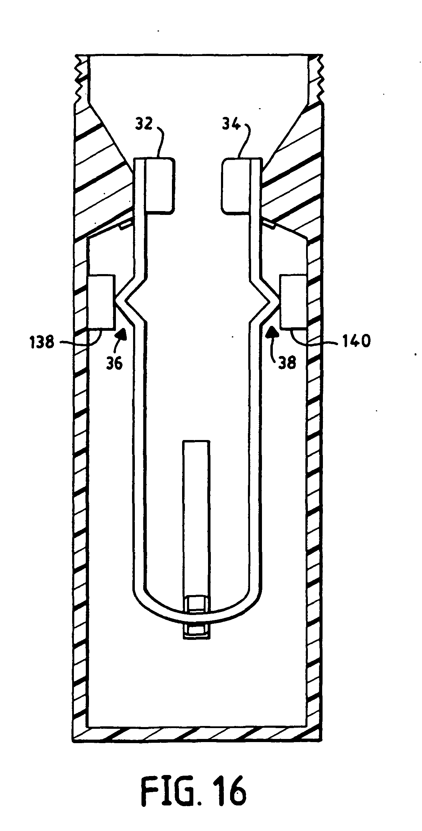

In Fig. 15, the operator has continued to push the knob 104 from the open end 72. As the protrusions 36, 38 pass the bottom of the throat surface 78, they trigger the timing circuit 130 by electrically connecting the start panels 134, 136. As the escapement 20 continues away from the open end 72, the mating surfaces 64, 66 remain held together by the viscoelasticity of the saliva sample on the mating surfaces 64, 66. In Fig. 16, the escapement 20 has reached the end of its travel. Eventually, the force of the spring 52 overcomes the viscoelasticity of the saliva sample, and the mating surfaces 64, 66 separate. When the mating surfaces 64, 66 separate, the protrusions 36, 38 electrically connect the stop panels 138, 140, signaling the timing circuit 130 to discontinue timing and to indicate the result of the measurement.

The escapement 20 is removable for disposal. To remove the escapement 20, the knob 104 is pushed back to the end of the slot 90 nearest the open end 72. Then the fork 100 is pulled radially out of the sheath 12 by the knob 104, and the escapement 20 is manually removed and disposed of.

Single-Prong Instrument Embodiment

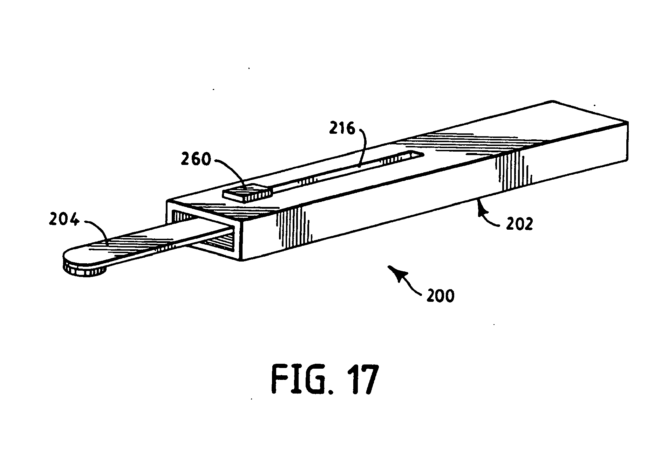

Fig. 17 shows the single-prong embodiment of the instrument of the present invention 200. In contrast to the first embodiment of Figs. 5-16, it is a substantially integral construction.

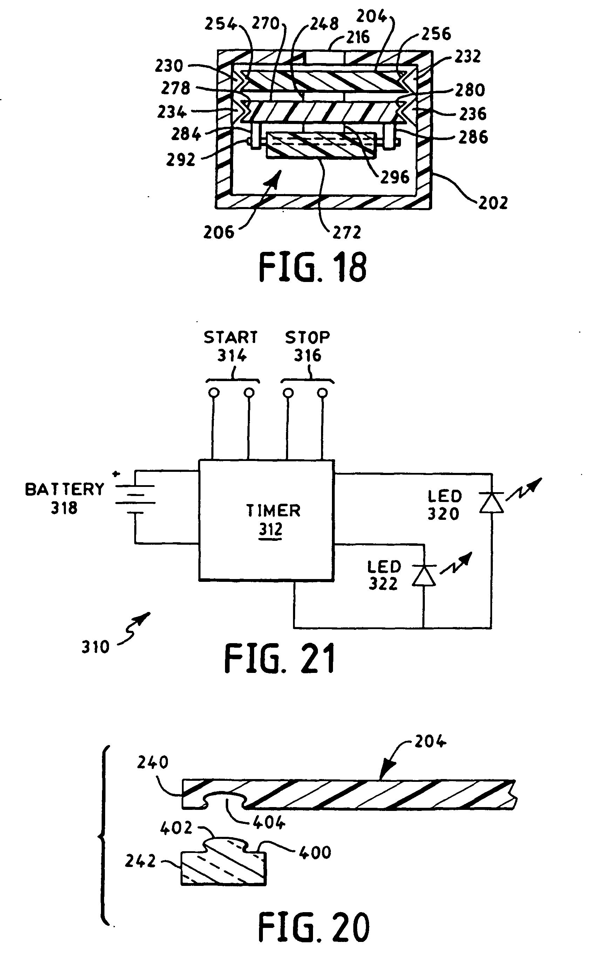

Figs. 18 and 19 show a front cross-sectional view and a side cross-sectional view, respectively. This embodiment 200 has three basic components, the sheath 202, the upper escapement portion 204, and the lower escapement portion 206.

The sheath 202 is composed of a rigid plastic and is approximately rectangular in shape and hollow. It has a height of about 25 mm, a width of about 30 mm, a length of about 130 mm, and a wall thickness of about 2 mm. One end of the sheath has an opening 212 through which the upper escapement portion 204 extends and retracts, as described below. The top wall of the sheath 214 has a longitudinal slot 216 that is approximately 90 mm long and 5 mm wide, the purpose of which is described below. Extending upwardly from the bottom wall 218 is a wedge 220. The leading surface of the wedge 222 slopes at an angle of about 45° to the bottom wall 218 and the trailing surface 224 extends downwardly to the bottom wall 218 away from the opening 212 at an angle of approximately 45°. The wedge 220 has a height of about 10 mm.

Projecting from the two long side walls 226, 228 near to and parallel with the upper wall 214 is a pair of upper rails 230, 232. Below the upper rails 230, 232 is a pair of lower rails 234, 236. In cross-section, all of the rails 230, 232, 234, 236 have a shape like a sideways "V". The purpose of the rails 230, 232, 234, 236 is described below.

The upper escapement portion 204 is a long, thin, rectangular-shaped solid that is composed of a slightly flexible plastic or composite. It is approximately 75 mm long, 25 mm wide, and 2 mm thick. Attached to the lower surface of the outer extremity of the upper escapement portion 240 is an upper plate 242, which is detailed below. In one embodiment, the upper plate 242 is attached to the upper escapement portion 240 by a substantially waterproof adhesive. In another embodiment, shown in Fig. 20, the upper plate 242 is attached by a snap mechanism. The rear of the upper plate 400 has a cylindrical protrusion 402 where the outer end is slightly larger in diameter than the inner end. In the lower surface of the end of the upper escapement portion 240 is a mating hole 404. The upper plate is attached to the upper escapement portion 204 by pressing the protrusion 402 into the hole 404 until it snaps into place. In a third embodiment, the upper plate 242 is integrally formed with the upper escapement portion 204. The upper plate 242 has a mating surface 262, which is detailed below.

The long edges of the upper escapement portion 204 are adapted by V-shaped indentations 254, 256 to mate with the sheath upper rails 230, 232. The upper rails 230, 232 and the indentations 254, 256 permit the upper escapement portion 204 reciprocate longitudinally within the sheath 202. Extending from the upper surface of the upper escapement portion 258 is a knob 260. The knob 260 is adapted to extend through the slot 216 and provides for manual reciprocation of the upper escapement portion 204 within the sheath 202. Extending downwardly near the center of the upper escapement portion 204 is a push tab 244 and extending downwardly from the inner extremity of the upper escapement portion 246 is a return tab 248. The tabs 244, 248 extend at least below the lower rails 234, 236 when the upper escapement portion 204 is installed on the upper rails 230, 232.

The lower escapement portion 206 has a shuttle 270 and an arm 272. The shuttle 270 is a rectangular solid that is composed of a rigid plastic. It is approximately 25 mm long, 25 mm wide, and 2 mm thick. The long edges of the shuttle 270 are adapted by V-shaped indentations 278, 280 to mate with the sheath lower rails 234, 236. The lower rails 234, 235 and the indentations 278, 280 permit the shuttle 270 reciprocate longitudinally within the sheath 202. When installed in the sheath 202, the shuttle 270 is located between the push tab 244 and return tab 246 of the upper escapement portion 204. Extending downwardly from the rear end of the shuttle 282 and adjacent to the long edges 274, 276 are a pair of ears 284, 286 to which the arm 272 is attached, as described below.

The arm 272 is a long, thin, rectangular-shaped solid that is composed of a slightly flexible material. It is approximately 50 mm long, 20 mm wide, and 2 mm thick. Attached to the outer extremity of the arm 288 is a lower plate 290. In one embodiment, the lower plate 290 is attached to the arm 272 by a substantially waterproof adhesive. In another embodiment, the lower plate 290 is attached to the arm 272 by a snap mechanism in the same manner as the upper plate 242 described above and in Fig. 20. In a third embodiment, the lower plate 290 is integrally formed with the arm 272. The lower plate 290 has a mating surface 264, which is detailed below. The arm 272 is pivotally attached to the shuttle 270 by an axle 292 that extends through holes in the ears 284, 286 and an edge-to-edge hole through the arm 272 near the inner extremity 294.

Located between the shuttle 270 and the arm 272 is a spring 296. The spring 296 can be a U type spring, as shown in Fig. 18, or a coil-type spring. The spring 296 forces the arm 272 away from the shuttle 270 about the pivot point created by the axle 292.

Located in the rear end of the sheath 300 are two holes 302, 304. Mounted within these holes 302, 304 are two LEDs 320, 322 for indicating the result of the test performed by the device. The radiating surfaces of the LEDs face outside the sheath 202.

Within the sheath 202 is a timing circuit 310, a schematic diagram of which is shown in Fig. 21. The timing circuit 310 is composed of a timer 312, a start switch 314, a stop switch 316, a battery 318, and the two LEDs 320, 322. When the start switch 314 is activated as described below, the timer 312 begins timing. When the stop switch 316 is activated as described below, the timer 312 stops timing. The timer 312 then compares the elapsed time to a predetermined value, and if the elapsed time is greater, it energizes one of the LEDs 320 momentarily. Otherwise it energizes the other LED 322 momentarily. The battery 318 supplies electrical power to the timing circuit 310.

In an alternative configuration, the timer 312 and battery 318 are mounted on the outside of the sheath 202 in order to be more easily accessible.

Figs. 19 and 22-25 detail, in cross-section, the internal operation of the single-prong embodiment 200. Initially, as in Fig. 19, the knob 260 is pushed to the end of the slot 216 nearest the sheath opening 212 so that the upper plate 242 is extending outwardly from the opening 212. Because of the force of the spring 296, the outer extremity end of the arm 288 is in contact with the bottom wall 218.

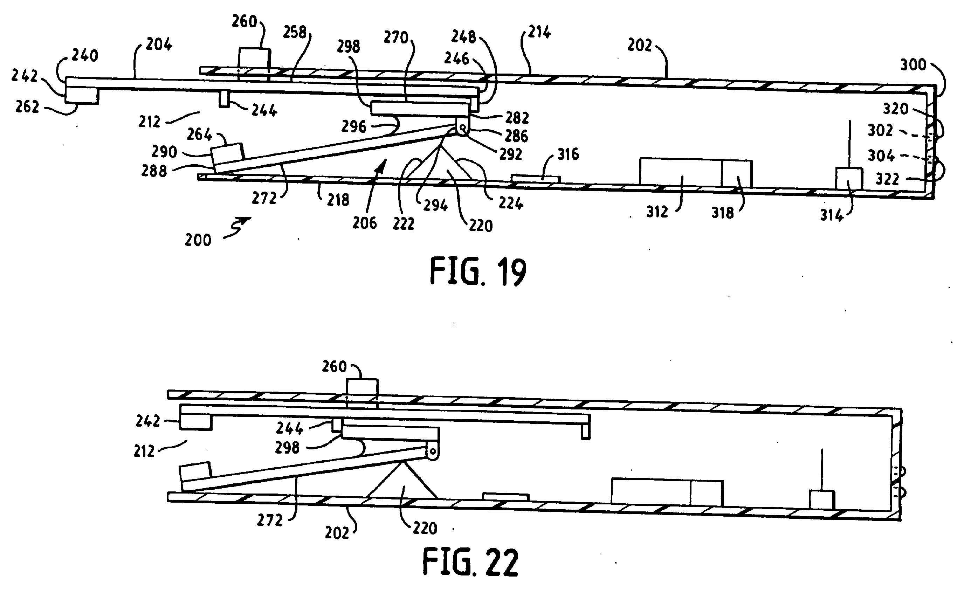

As in Fig. 22, the operator has pushed the knob 260 away from the opening 212 a short distance. Pushing the knob 260 causes the upper plate 242 to be withdrawn into the sheath 202 through the opening 212. At substantially the same moment that the upper plate 242 is completely withdrawn into the sheath 202, the push tab 244 makes contact with the outer extremity of the shuttle 298 and the top edge of the wedge 220 makes contact with the arm 272.

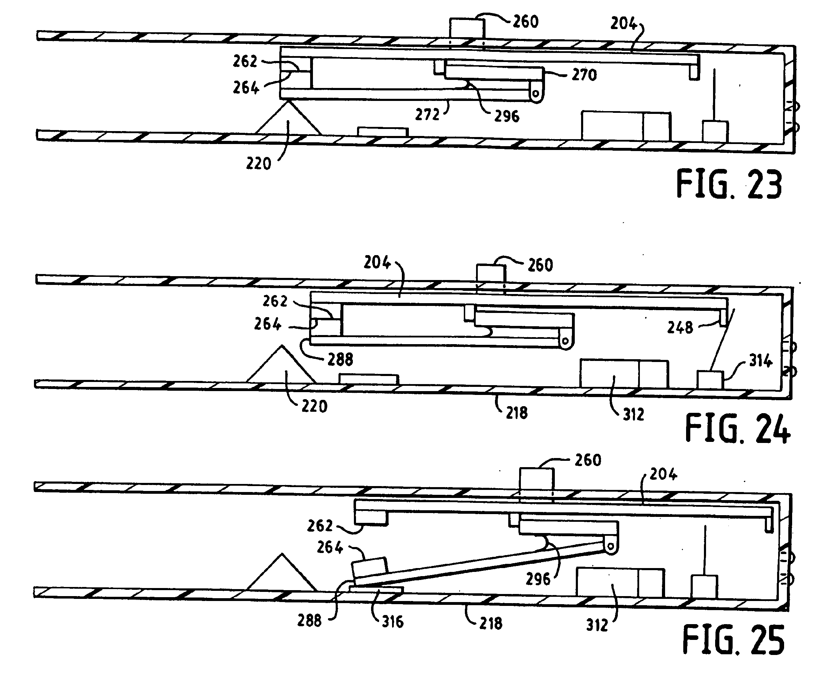

As in Fig. 23, the operator has continued to push the knob 260 away from the opening 212. The push tab 244 is pushing the shuttle 270 along with the upper escapement portion 204. The action of the wedge 220 on the arm 272 acts as a camming mechanism to overcome the force of the spring 296 and cause the arm 272 to pivot upwardly until the mating surfaces 262, 264 make contact. This action causes the upper escapement portion 204 and the arm 272 to deform away from each other. The amount of force holding the mating surfaces 262, 264 and, as a consequent, compressing the saliva sample, is related to the amount of deformation and preferably is approximately 15 grams.

As in Fig. 24, the operator has continued to push the knob 260 away from the opening 212. As the outer extremity of the arm 288 passes the top edge of the wedge 220, the return tab 248 activates the start switch 314 extending from the bottom wall of the sheath 218. The start switch 314 triggers the timer 312. As the upper escapement portion 204 continues rearwardly, the mating surfaces 262, 264 are no longer being forced together by the wedge 220, but remain held together by the viscoelasticity of the saliva sample on the mating surfaces 262, 264. In Fig. 25, the upper escapement portion 204 has reached the end of its travel. Eventually, the force of the spring 296 overcomes the viscoelasticity of the saliva sample, and the mating surfaces 262, 264 separate. When this happens, the outer extremity of the arm 288 stops the timer 312 by activating the stop switch 316 located on the bottom wall 218.

The plates 242, 290 are removable in order to replace them. To remove the plates 242, 290, the knob 260 is pushed back to the end of the slot 216 nearest the opening 212, extending the outer extremity of the upper escapement portion 240 from the opening 212. This allows access to the upper plate 242 for removal. The upper plate 242 is removed by prying it out of the snap hole 404. The lower plate 290 is accessible through the opening 212 and is removed in the same manner as the upper plate 242.

The Plates

The plates of both the dual-prong embodiment 10 and single-prong embodiment 200 are substantially the same. Each has a mating surface. When the mating surfaces are in contact, as described above, the area of contact is substantially the entire face area of the plates. Preferably, the plates are approximately round with a face diameter of between 0.5 and 0.7 centimeters (cm), which is a face area of approximately 0.2 to 0.4 cm$^2$. The plates are between 0.2 and 0.5 cm thick.

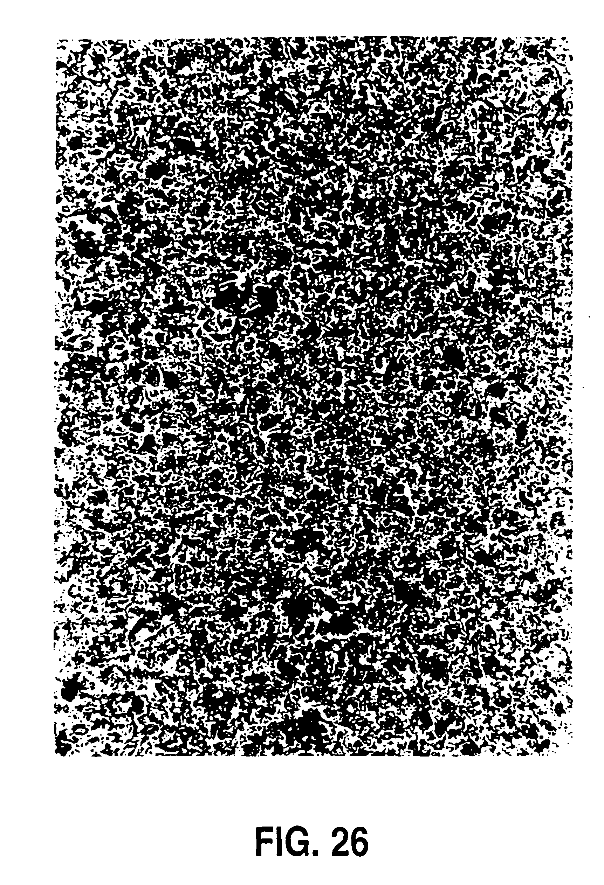

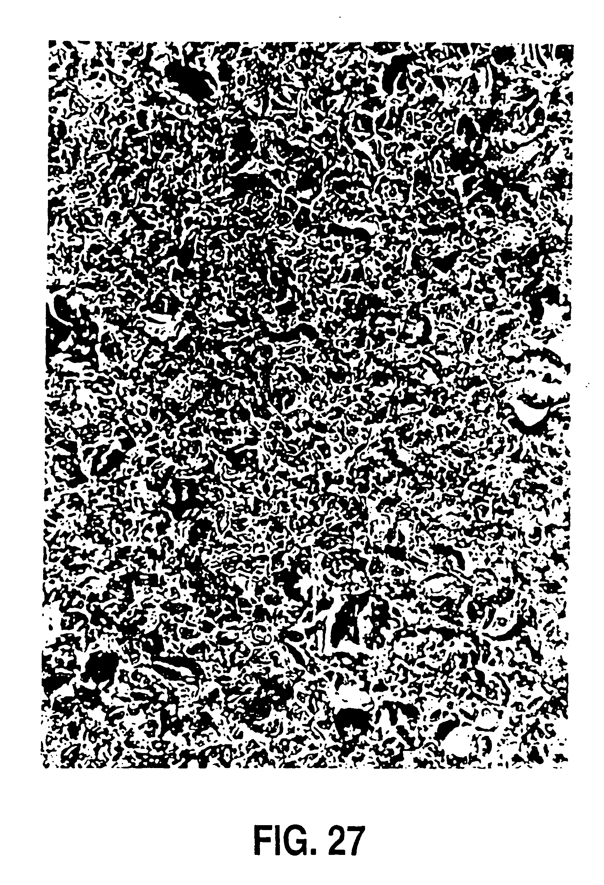

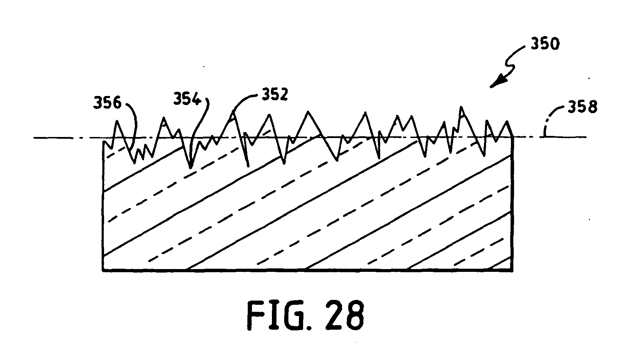

Fig. 26 is a low-power (10X) microphotograph, Fig. 27 is a high-power (40X) microphotograph, and Fig. 28 is a greatly enlarged cross-section of a preferred roughened plate surface 350. As can be seen, the plate surface 350 is characterized by a random distribution of irregularly-shaped peaks 352 and valleys 354. There are two basic parameters that are important in characterizing the plate surface 350 when used in an instrument for measuring saliva viscoelasticity. The first of these parameters is the average depth of the valleys, as measured from the plane defined by the tops of the peaks 352. The preferred range of this average is from 10 picometers (pm) to 100 micrometers ($\mu$m), and the most preferred range is from 50 $\mu$m to 80 $\mu$m.

The second parameter is the amount of valley area 356, the sum of the surface area of the valley walls below one half of the average depth of the valleys 358, relative to the total surface area. The preferred range of valley area 356 is from 35% to 65% of the total surface area, and the most preferred range is from 45% to 55%.

The preferred amount of roughening will double the smooth size of the surface area. This is achieved by having an average depth of approximately 65 $\mu$m and a valley area of approximately 50%.

If the average depth of the valleys 354 is too shallow, such as less than 10 pm, the plate surface 350 will be too smooth and will not work adequately for two reasons. First, the area of the plate surfaces 350 will be so small that the saliva sample will not adhere with a force greater than the cohesion of the saliva sample. As explained above, if the force of adhesion is smaller than the force of cohesion of the saliva sample, the saliva sample will separate from the plate surfaces 350 before it fractures.

Second, one effect of very smooth surfaces of like material is that they will adhere to each other on a molecular level, requiring a very large separation force to pull the surfaces apart. The separation force would be too large to be practical in actual usage because sublingual saliva has a very low viscoelasticity, which requires a finely calibrated separation force for an accurate measurement. And because it is more difficult to calibrate a large force to the same absolute accuracy as a small force, the larger force needed to separate smooth surfaces would be more difficult to calibrate to a particular value than the small force needed to separate roughened surfaces, resulting in a less accurate measurement of viscosity.

If the average depth of the valleys 354 is too great, such as greater than 100 $\mu$m, or the ratio of valley area 356 to total area is too large, such as greater than 65%, the surface will also not work adequately because the saliva sample would spread into the deep or large valleys, leaving the saliva sample remaining outside the valleys 354 too small for an accurate measurement. If the saliva sample is too small, it will not cover the entire area of the plate surfaces 350, resulting in an inaccurate value for the fracturing surface area, and rendering the calculated viscoelasticity inaccurate.

If the ratio of valley area 356 to total area is too low, such as less than 35%, the plate surface 350 will also not work adequately because the area of the plate surfaces 350 will be so small that the saliva sample will not adhere with a force greater than the cohesion of the saliva sample. As explained above, if the force of adhesion is smaller than the force of cohesion of the saliva sample, the saliva sample will separate from the sample surfaces 350 before it fractures.

The plates are composed of glass or a plastic. When glass, the face is ground to the above-described roughened surface. When plastic, the surface is etched into a mold from which the plate is formed, eliminating the extra grinding step. In the dual-prong embodiment 10, the plates are preferably composed of plastic, because then the escapement 20 can be molded with the plates as integral components. In the single-prong embodiment 200, the plates are preferably either glass or plastic. Since they are preferably separable from the upper escapement portion 204 and the arm 272 and are not integrally formed with those components, they do not have to be composed of the same material as the upper escapement portion 204 and the arm 272.

The plates are intended to be single-use disposable items. The faces can be used for one measurement only because the previous saliva sample will dry in the crevices of the face, causing a subsequent measurement to be invalid since the new saliva sample cannot adhere properly to the face.

Determination of Fertile Period

The elapsed time measured by the timing circuit 130, 310 is the time it takes for the force of the spring 52, 296 to overcome the tendency of the saliva sample to remain intact, the force of cohesion. The viscoelasticity of the saliva sample is directly related to this time measurement by equation (1) above.

In the dual-prong embodiment, the preferred size of the plate faces 64, 66 is between about 0.2 cm$^2$ and 0.4 cm$^2$. However, in order for the saliva sample to fracture before it separates from the plate faces 64, 66, the plate faces 64, 66 are roughened to about twice the nominal surface area, namely from about 0.4 cm$^2$ to about 0.8 cm$^2$. A predetermined constant pressure is applied between the plate faces 64, 66 for a minimum period of time of approximately 2 to 4 seconds when the protrusions 36, 38 are within the throat 82. This pressure compresses and extrudes the saliva sample between the plate faces 64, 66. After the protrusions 36, 38 are beyond the throat 82, the pressure of the spring 52 acts to fracture the saliva sample. The spring force is set to about 0.001 dy, giving a shear stress of between about 0.0013 dy/cm$^2$ and 0.0025 dy/cm$^2$. The shear stress is divided by the inverse of the amount of time measured by the timer 130 to arrive at the viscoelasticity of the saliva sample. For example, if the shear stress is 0.0017 dy/cm$^2$ and the separation time is measured as 30 seconds, the viscoelasticity is calculated as 0.051 P or 5.1 cP.

In the single-prong embodiment, the preferred size of the plate faces 262, 264 is between about 0.2 cm$^2$ and 0.4 cm$^2$. However, in order for the saliva sample to fracture before it separates from the plate faces 262, 264, the plate faces 262, 264 are roughened to about twice the nominal surface area, namely from about 0.4 cm$^2$ to about 0.8 cm$^2$. A predetermined constant pressure is applied between the plate faces 262, 264 for a minimum period of time of approximately 2 to 4 seconds when the arm 272 is pushed up by the wedge 220. This pressure compresses and extrudes the saliva sample between the plate faces 262, 264. After the front end of the arm 288 is beyond the wedge 220, the pressure of the spring 296 acts to fracture the saliva sample. The spring force is set to about 0.001 dy, giving a shear stress of between about 0.0013 dy/cm$^2$ and 0.0025 dy/cm$^2$. The shear stress is divided by the inverse of the amount of time measured by the timer 310 to arrive at the viscoelasticity of the saliva sample. For example, if the shear stress is 0.0017 dy/cm$^2$ and the separation time is measured as 30 seconds, the viscoelasticity is calculated as 0.051 P or 5.1 cP.

The viscoelasticity of sublingual saliva relates to the fertile period. The viscoelasticity falls over a period of from 2 to 4 days until about 16 to 24 hours prior to ovulation and then rises over a period of from 1 to 2 days. Note that a viscoelasticity of 5.1 cP shows that the women is either about 2 days prior to ovulation or immediately after ovulation.

**OPERATION**

Each of the two above-disclosed embodiments operate in essentially the same manner. However, there is some variation, so the operation of each is described separately.

Dual-Prong Embodiment

The dual-prong embodiment 10 is designed so that the escapement 20 is disposable. Prior to usage, a new escapement 20 is acquired and inserted into the sheath 12 by the following method: (1) push the knob 104 to the end of the slot 90 near the top end of the sheath 72, (2) pull the knob 104 out of the sheath 12 against the coil spring 116 until it stops and hold the knob 104 out, (3) insert the escapement 20 into the top end 72 until the protrusions 36, 38 are resting on the upper throat surface 76, and (4) release the knob 104, allowing the coil spring 116 to pull the fork 100 back into the sheath 12. During this insertion process, care must be taken to avoid contaminating the plates 32, 34 with any foreign substance, as such contamination will invalidate the measurement.

After the escapement 20 is in place within the sheath 12, the plates 32, 34 are dipped into the mouth in order to retrieve a sample of sublingual saliva from the saliva pool under the tongue. The saliva must cover substantially the entire face of the plates 64, 66. After taking the saliva sample, the bottom end of the sheath 12 is inserted into the opening in the stand 18. Then the knob 104 is pushed the full distance of the slot 90 in a substantially smooth motion. If the smooth motion is not maintained or the knob 104 is not pushed the full distance of the slot 90, an invalid measurement will result. After a period of time, one of the LEDs 144, 146 will illuminate momentarily. If one LED 144 illuminates, it is between approximately 48 hours and 5 hours before ovulation. If the other LED 146 illuminates, it is not within that time period before ovulation.

After a measurement is taken, the escapement 20 must be discarded, since it can only be used for one measurement. To remove the escapement 20, push the knob 104 back the full distance of the slot 90, pull the knob 104 out of the sheath 12, and pull the escapement 20 from the sheath 12.

In alternate embodiment, the entire instrument 10 is disposable. In this embodiment, the instrument 10 is acquired with the escapement 20 already installed. After being used for a single measurement, the instrument 10 is disposed of properly. Single-Prong Embodiment

The single-prong embodiment 200 is designed so that the plates 242, 290 are disposable. Prior to usage, a new pair of plates 242, 290 is acquired and installed onto the outer end of the upper escapement portion 240 and the arm 288 by snapping the upper plate 242 it the hole 404 in the upper escapement portion 204 and by snapping the lower plate 290 in the hole in the arm 272. During this installation process, care must be taken to avoid contaminating the plates 242, 290 with any foreign substance, as such contamination will invalidate the measurement.

After the plates 242, 290 are installed, the upper plate 242 is dipped into the mouth under the tongue in order to retrieve a sample of sublingual saliva. The saliva must cover substantially the entire face of the plate 262. After taking the saliva sample, the knob 260 is pushed the full distance of the slot 216 in a substantially smooth motion. If the smooth motion is not maintained or the knob 260 is not pushed the full distance of the slot 216, an invalid measurement will result. After a period of time, one of the LEDs 320, 322 will illuminate momentarily. If one LED 320 illuminates, it is between approximately 48 hours and 5 hours before ovulation. If the other LED 322 illuminates, it is not within that time period before ovulation.

After a measurement is taken, the plates 242, 290 must be discarded, since it can only be used for one measurement. To remove the plates 242, 290, push the knob 260 back the full distance of the slot 216 and pry the upper plate 242 from the hole 404. Remove the lower plate 290 by prying it from the arm hole.

In alternate embodiment, the entire instrument 200 is disposable. In this embodiment, the instrument 10 is acquired with the plates 242, 290 already installed. After being used for a single measurement, the instrument 200 is disposed of properly.

**Claims**

1. An instrument for determining female fertile period by measuring the viscoelasticity of saliva, said device comprising:

 (a) an elongated sheath having a length along an axis and an opening at one extremity;
 (b) a reciprocable escapement including a pair of elongated arms disposed approximately parallel to said axis, said arms having inner extremities within said sheath remote from said opening and outer extremities with mating surfaces;
 (c) said escapement including a spring bias urging the separation of said mating surfaces from each other;
 (d) said arms being mounted for relative movement of said mating surfaces among first relational positions at which said mating surfaces are separated, second relational positions at which said mating surfaces are in contact, and third relational positions at which said mating surfaces are free to separate under said spring bias;
 (e) a control operatively connected to said escapement for optionally placing said mating surfaces into said first relational positions, said second relational positions, and said third relational positions;
 (f) at least one of said mating surfaces being free to collect said saliva when said mating surfaces are in said first relational positions;
 (g) said mating surfaces being constrained to compress said saliva therebetween when said mating surfaces are in said second relational positions;
 (h) a timer for measuring the time elapsed for separation of said surfaces when said surfaces are in said third relational positions; and

(i) said mating surfaces adapted to retain said saliva in a manner that causes said saliva to internally fracture before said saliva overcomes its adhesion to said mating surfaces when in said third relational positions.

2. The instrument of claim 1 wherein said escapement is removable for disposal and replacement.

3. The instrument of claim 1 wherein said spring bias is produced at a junction at said inner extremities of said arms.

4. The instrument of claim 1 wherein said sheath includes a camming mechanism for moving said mating surfaces from said first relational position to said second relational positions and from said second relational positions to said third relational positions.

5. The instrument of claim 4 wherein said camming mechanism includes opposing elbows on said arms and a throat within said sheath aligned with said axis, whereby the movement of said elbows into said throat causes said mating surfaces to move from said first relational positions to said second relational positions and the movement of said elbows out of said throat causes said mating surfaces to move from said second relational positions to said third relational positions.

6. The instrument of claim 1 wherein said timer is an electronic circuit.

7. The instrument of claim 1 wherein said timer includes a visual indicator.

**Patentansprüche**

1. Gerät zur Bestimmung der weiblichen Fruchtbarkeitsperiode durch Messen der Viskoelastizität von Speichel, **gekennzeichnet durch**

a) eine längliche Hülle mit einer Längserstreckung in einer Achse und einer Öffnung an einer Extremität;
b) eine hin- und her bewegliche Hemmvorrichtung mit einem Paar von länglichen Armen, die etwa parallel zur Achse angeordnet sind, wobei die Arme innere Extremitäten innerhalb der Hülle entfernt von der Öffnung und äußere Extremitäten mit zusammengehörigen Oberflächen aufweisen;
c) die Hemmvorrichtung eine Federvorspannung aufweist, die das Trennen der zusammengehörigen Oberflächen voneinander bewirkt;
d) die Arme für eine relative Bewegung der zusammengehörigen Oberflächen zwischen ersten Relativpositionen, bei denen die zusammengehörigen Oberflächen getrennt sind, zwischen zweiten Relativpositionen, bei denen die zusammengehörigen Oberflächen in Kontakt miteinander stehen, und zwischen dritten Relativpositionen, bei denen die zusammengehörigen Oberflächen sich frei unter der Federvorspannung trennen können, befestigt sind;
e) eine Steuerung, die mit der Hemmvorrichtung operativ verbunden ist, um die zusammengehörigen Oberflächen in die ersten Relativpositionen, die zweiten Relativpositionen und die dritten Relativpositionen nach Belieben zu bringen;
f) mindestens eine der zusammengehörigen Oberflächen frei ist, um den Speichel zu sammeln, wenn die zusammengehörigen Oberflächen in den ersten Relativpositionen stehen,
g) die zusammengehörigen Oberflächen so festgelegt sind, dass sie den Speichel dazwischen komprimieren, wenn die zusammengehörigen Oberflächen in den zweiten Relativpositionen stehen;
h) eine Zeitsteuervorrichtung zur Messung der Zeitdauer, die zur Trennung der Oberflächen verstrichen ist, wenn die Oberflächen in den dritten Relativpositionen stehen; und
i) die zusammengehörigen Oberflächen in der Lage sind, den Speichel so zurückzuhalten, dass er intern abreißt, bevor er seine Adhäsion mit den zusammengehörigen Oberflächen überwindet, wenn er sich in den dritten Relativpositionen befindet.

2. Gerät nach Anspruch 1, bei dem die Hemmvorrichtung für die Beseitigung und Auswechslung entfernbar ist.

3. Gerät nach Anspruch 1, bei dem die Federvorspannung an einer Verbindungsstelle an den inneren Extremitäten der Arme erzeugt wird,

4. Gerät nach Anspruch 1, bei dem die Hülle einen Steuerungsmechanismus zur Bewegung der zusammengehörigen Oberflächen aus den ersten Relativpositionen in die zweiten Relativpositionen und von den zweiten Relativpositionen in die dritten Relativpositionen aufweist.

**5.** Gerät nach Anspruch 4, bei der der Steuerungsmechanismus entgegengesetzte Ellbogen an den Armen und eine Engstelle innerhalb der Hülle, die mit der Achse ausgerichtet ist, aufweist, wobei die Bewegung der Ellbogen in die Engstelle bewirkt, dass die zusammengehörigen Oberflächen sich aus den ersten Relativpositionen in die zweiten Relativpositionen bewegen, und die Bewegung der Ellbogen aus der Engstelle bewirkt, dass die zusammengehörigen Oberflächen sich von den zweiten Relativpositionen in die dritten Relativpositionen verschieben.

**6.** Gerät nach Anspruch 1, bei dem die Zeitsteuereinrichtung eine elektronische Schaltung ist.

**7.** Gerät nach Anspruch 1, bei dem die Zeitsteuerschaltung eine visuelle Anzeigevorrichtung aufweist.

**Revendications**

**1.** Instrument pour déterminer la période de fertilité de la femme en mesurant la viscoélasticité de la salive, ledit dispositif comprenant:

(a) un fourreau allongé ayant une longueur le long d'un axe et une ouverture à une extrémité;

(b) un échappement avec possibilité de va-et-vient comprenant une paire de bras allongés situés à peu près parallèlement audit axe, lesdits bras ayant les extrémités internes à l'intérieur dudit fourreau éloignées de ladite ouverture et les extrémités externes comportant des surfaces se correspondant;

(c) ledit échappement comprenant une déflection par ressort contraignant lesdites surfaces se correspondant à s'écarter l'une de l'autre;

(d) lesdits bras étant installés pour permettre le déplacement relatif desdites surfaces se correspondant entre les premières positions d'interrelation où lesdites surfaces se correspondant sont écartées, les secondes positions d'Interrelation où lesdites surfaces se correspondant sont en contact, et les troisièmes positions d'interrelation où lesdites surfaces se correspondant sont libres de s'écarter par l'effet de la déflection par ressort;

(e) une commande reliée de manière opérationnelle audit échappement pour positionner au choix lesdites surfaces se correspondant auxdites premières positions d'interrelation, auxdites secondes positions d'interrelation, et auxdltes troisièmes positions d'interrelation;

(f) au moins une desdites surfaces se correspondant étant libre pour récolter ladite salive quand lesdites surfaces se correspondant sont dans lesdites premières positions d'interrelation;

(g) lesdites surfaces se correspondant étant obligées de comprimer entre elles ladite salive quand lesdites surfaces se correspondant sont auxdites secondes positions d'interrelation;

(h) un chronomètre pour mesurer le temps écoulé pour la séparation desdites surfaces quand lesdites surfaces sont auxdites troisièmes positions d'interrelation; et

(I) lesdites surfaces se correspondant sont adaptées pour retenir ladite salive de manière à provoquer la rupture interne de ladite salive avant que ladite salive ne vainque son adhérence auxdites surfaces se correspondant quand positionnées auxdltes troisièmes positions d'interrelation.

**2.** Instrument selon la revendication 1 dans lequel ledit échappement peut être enlevé pour mise au rebut et remplacement.

**3.** Instrument selon la revendication 1 dans lequel ladite déflection par ressort se produit à la jonction desdites extrémités intérieures desdits bras.

**4.** Instrument selon la revendication 1 dans lequel ledit fourreau comprend un mécanisme à cames pour déplacer lesdites surfaces se correspondant depuis lesdites premières positions d'interrelation jusqu'auxdites secondes positions d'interrelation et depuis lesdites secondes positions d'interrelation jusqu'auxdites troisièmes positions d'interrelation.

**5.** Instrument selon la revendication 4 dans lequel ledit mécanisme à cames comprend des coudes s'opposant sur lesdits bras et un rétrécissement à l'intérieur dudit fourreau aligné avec ledit axe, par quoi le déplacement desdits coudes à l'intérieur dudit rétrécissement provoque le déplacement desdites surfaces se correspondant depuis lesdites premières positions d'Interrelation jusqu'auxdltes secondes positions d'interrelation et le déplacement desdits coudes en dehors dudit rétrécissement provoque le déplacement desdites surfaces se correspondant depuis lesdites secondes positions d'interrelation jusqu'auxdites troisièmes positions d'interrelation.

**6.** Instrument selon la revendication 1 dans lequel ledit chronomètre est un circuit électronique.

**7.** Instrument selon la revendication 1 dans lequel ledit chronomètre comprend un indicateur de visualisation.

FIG.1

FIG.2

FIG.3

FIG.4

FIG. 5

FIG. 6

FIG. 7

FIG. 8

FIG. 9

START STOP
134 136 138 140

TIMER
132

FIG. 10

FIG. 11

FIG.12

FIG. 13

FIG. 14

FIG.15

EP 1 413 254 B1

FIG. 16

22

FIG. 17

FIG. 18

FIG. 21

FIG. 20

FIG. 19

FIG. 22

EP 1 413 254 B1

EP 1 413 254 B1

FIG. 23

FIG. 24

FIG. 25

FIG. 26

FIG. 27

**FIG. 28**